# EUROPEAN PATENT APPLICATION

(11) **EP 3 843 103 A1**
(43) Date of publication of application: **30.06.2021**
(21) Application number: 20216226.9
(22) Date of filing: 21.12.2020
(51) Int. Cl.: G16H 20/10, G16H 40/20

(54) **SYSTEMS AND METHODS FOR INCREASING ADHERENCE FOR MEDICAL DEVICES**

(30) Priority: 25.12.2019 US 201962953553 P
(71) Applicant: Softimize Ltd., 5624134 Yehud-Monosson (IL)
(72) Inventor: Vinograd, Guy, Yehud (IL); Last, Maor, Petah Tikva (IL); Rotem, Joel, Kfar Sirkin (IL)
(74) Representative: Pearl Cohen Zedek Latzer Baratz UK LLP

(57) **Abstract**

A system and method for detecting adherence to a medical device including obtaining a usage schedule of the medical device, obtaining device status data of the medical device, and calculating a predicted adherence score based on the usage schedule and the device status data. The predicted adherence may be calculated using a machine learning algorithm that may be patient specific, device type specific, or disease specific.

## Description

### FIELD OF THE INVENTION

The present invention relates generally to technology increasing adherence for medical devices, using prescription data, device usage data and general device data.

### BACKGROUND

A physician may prescribe a medical device to a patient as part of a treatment. However, many patients fail to use the medical device in the home setting. Some users adhere to the medical device, e.g., use the medical device according to the prescription. However, often users do not adhere to the medical device. For example, users may only partially follow the prescription, may not use the device at all, may use the device differently than prescribed, or may even over-use the device.

The problem of non-adherence to medical devices may be much more severe than medication non-adherence, due to the more difficult process of using some of the medical devices compared to the quick and easy process of taking a pill. Furthermore, there are some hints that adherence problems may be a result of usability problems of medical devices.

Medication adherence applications typically ask the patient if they took the medication or not. Thus, the applications typically have no way of objectively knowing whether the data is correct or not. In fact, adherence applications may make the adherence problem even worse, because they require application adherence in addition to medication adherence.

Therefore, a method for detecting true adherence and for increasing adherence to medical devices is required.

### SUMMARY

According to embodiments of the invention, a system and method for detecting adherence to a medical device may include obtaining a usage schedule of the medical device; obtaining device status data of the medical device; and calculating a predicted adherence score based on the usage schedule and the device status data.

According to embodiments of the invention, the status data may include at least one of: battery status, error logs and location of the medical device.

According to embodiments of the invention, the status data may include battery charge status, and embodiments of the invention may include calculating the predicted adherence score based on the battery charge status.

According to embodiments of the invention, the status data may include location of the medical device, and embodiments of the invention may include calculating the predicted adherence score based on the geographic location of the medical device.

Embodiments of the invention may include providing an alert in case the predicted adherence score exceeds a threshold.

According to embodiments of the invention, calculating the predicted adherence may be performed using a machine learning algorithm.

According to embodiments of the invention, the machine learning algorithm may be one of patient specific, device type specific, or disease specific.

According to embodiments of the invention, detecting usage data of the medical device; calculating an actual adherence score by matching the usage schedule and the usage data; and performing a root cause analysis for detecting the reason for non-adherence by correlating the actual adherence score and the device status data.

Embodiments of the invention may include calculating the actual adherence score based on allowed deviations from the usage schedule.

Embodiments of the invention may include providing a notification about non-adherence including the root cause of the non-adherence.

Embodiments of the invention may include providing a recommendation for a change in the design of the device based on the root cause of the non-adherence.

### BRIEF DESCRIPTION OF THE DRAWINGS

The subject matter regarded as the invention is particularly pointed out and distinctly claimed in the concluding portion of the specification. Embodiments of the invention, however, both as to organization and method of operation, together with objects, features and advantages thereof, may best be understood by reference to the following detailed description when read with the accompanied drawings. Embodiments of the invention are illustrated by way of example and not limitation in the figures of the accompanying drawings, in which like reference numerals indicate corresponding, analogous or similar elements, and in which:
Fig. 1 schematically illustrates a system, according to embodiments of the invention;
Fig. 2 schematically illustrates medical device, according to embodiments of the invention;
Fig. 3 is a flowchart of a method for calculating predicted adherence, according to embodiments of the invention;
Fig. 4 is a flowchart of a method for adjusting reimbursement rate and purchase rate of medical devices based on adherence level, according to embodiments of the invention; and
Fig. 5 illustrates an example computing device according to an embodiment of the invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION

In the following description, various aspects of the present invention will be described. For purposes of explanation, specific configurations and details are set forth in order to provide a thorough understanding of the present invention. However, it will also be apparent to one skilled in the art that the present invention may be practiced without the specific details presented herein. Furthermore, well known features may be omitted or simplified in order not to obscure the present invention.

Although some embodiments of the invention are not limited in this regard, discussions utilizing terms such as, for example, "processing," "computing," "calculating," "determining," "establishing", "analyzing", "checking", or the like, may refer to operation(s) and/or process(es) of a computer, a computing platform, a computing system, or other electronic computing device that manipulates and/or transforms data represented as physical (e.g., electronic) quantities within the computer's registers and/or memories into other data similarly represented as physical quantities within the computer's registers and/or memories or other information transitory or non-transitory or processor-readable storage medium that may store instructions, which when executed by the processor, cause the processor to execute operations and/or processes. Although embodiments of the invention are not limited in this regard, the terms "plurality" and "a plurality" as used herein may include, for example, "multiple" or "two or more". The terms "plurality" or "a plurality" may be used throughout the specification to describe two or more components, devices, elements, units, parameters, or the like. The term "set" when used herein may include one or more items unless otherwise stated. Unless explicitly stated, the method embodiments described herein are not constrained to a particular order or sequence. Additionally, some of the described method embodiments or elements thereof can occur or be performed in a different order from that described, simultaneously, at the same point in time, or concurrently.

The Internet of medical things (IoMT), also referred to as the Internet of things (IoT) in healthcare, relates to connecting medical devices to servers and to cloud computing using networking technologies. Connected medical devices, e.g., medical devices that are connected to the internet, may provide an accurate online source of adherence data: the medical device itself. Data gathered from the medical device may provide accurate information regarding when and how much the patient uses the medical device. Furthermore, the connected medical device may be used to alert the patient or caregiver (e.g., anyone who treats the patient in practice or has an influence on the patient's behavior, including nurses, relatives, etc.) in case of non-adherence. While embodiments of the invention relate to medical devices, e.g., devices that are used for medical purposes such as diagnosing or a treating disease, embodiments of the invention are not limited in this regard and may be used with other devices that connect to the internet and require adherence.

Furthermore, embodiments of the invention may provide new correlations of root causes leading to non-adherence by gathering device data that is not directly related to adherence, such as firmware version, battery status, error logs, device location etc., and cross-referencing it with device usage by patient and the schedule dictated by the medical protocol. Those root causes may be used to increase the adherence by direct interactions with patients or their influencers (e.g., family, caregivers, payers), to determine reimbursement rate of payer to patient, to determine purchase rate of payer from medical device vendor, and ultimately, to allow medical device vendors to design next generation devices that avoid those root causes as much as possible.

Some embodiments of the invention may predict future adherence based on usage schedule and treatment data, including device status data, patient data and other information related to the medical treatment, and may provide an alert in case the predicted adherence score is low. Thus, embodiments of the invention may improve adherence by providing an alert to the relevant users (e.g., the patient or the caregiver) ahead of time, in case that non-adherence is likely. In addition, in some cases embodiments of the invention may provide the root cause for the low predicted adherence score and by that may enable the relevant user to perform a corrective action that would improve adherence. For example, in case that low predicted adherence is detected, and the root cause for the low predicted adherence is low charge level of the battery, the user may obtain the alert for predicted non-adherence together with the root cause, ahead of time. Thus, the user may charge the battery and use the device on time as prescribed.

According to embodiments of the invention, treatment data may include device data or device status data, including device general status data (data related to the medical device but not directly to the medical functionality of the device, e.g., battery status, location etc.), device medical data (e.g., information related to treatments and measurements provided to the patient), patient data and other information related to the medical treatment.

Examples of possible relations between device status data and adherence may include:

Battery charge status - non-adherence may be a result of a battery that is not charged when the device should be used. Embodiments of the invention may use the battery charge level to predict adherence levels. Embodiments of the invention may further provide alerts and notifications to charge the battery in case that the battery charge is low, and the device needs to be used.

Device specification - weight and dimensions of the medical device, as well as specifications of hardware modules (e.g., Bluetooth low energy (BLE), battery, Wi-Fi modules etc.) included in the medical device may influence device usability and adherence. Specifications of the hardware modules may include type of the hardware modules, manufacturers of the hardware modules, and internal specifications of the hardware modules. For example, if the device is big and heavy this may reduce usability and adherence.

Device usage scenario - the device usage scenario, e.g., wearable, implantable, stationary, may influence usability adherence. For example, if the device is wearable this may increase adherence.

Firmware version - a new firmware update may influence adherence, for example if the new firmware includes a bug.

Device configuration - configuration of certain parameters may influence adherence.

Location - the location of the device or the location of the patient may influence adherence. For example, if the device was lost or is for some other reason distant from the user this may have an effect on the location of the device and on adherence.

Failed attempts - adherence may decrease if a patient tried using the device and failed. According to embodiments of the invention failed attempts may be detected and used to predict adherence.

Log files - error logs files may indicate that the patient has trouble with using the device and therefore may be a predictor of non-adherence.

Other parameters, not necessarily related to the device, may influence adherence. For example, the caregiver attitudes, such as a level of involvement and satisfaction with the patient and/or with the treatment, including whether the caregiver conveys this satisfaction to the patient, may influence adherence. This information may be provided by for example a caregiver questionnaire, e.g., using a caregiver user device.

Known methods for improving adherence may include interventions by the health care provider, e.g., explaining and motivating the patient or his caregiver to adhere to the prescribed medical device. However, if a device was not well designed, e.g., if the device is complicated and inconvenient to use, there may be a very low upper limit to how much adherence can be improved by interventions. Embodiments of the invention may provide computing systems that may analyze adherence with relation to device parameters, and may provide insights or root causes to non-adherence that are related to the device parameters. Such insights may be later used by device designers and manufacturers to improve the device and increase usability and adherence.

Embodiments of the invention may use artificial intelligence and machine learning models to predict adherence per patient, per device, per device type, per disease or any other meaningful categorization, that may provide insights to adherence patterns and root causes for non-adherence.

Embodiments of the invention may be used to predict adherence to various medical devices including (but not limited to) heart implants requiring an external device, congestive heart failure (CHF) monitoring devices, sleep sensors, pill dispensers, drug delivery devices, physiotherapeutic shoes and other orthopedic rehabilitation devices, pregnancy monitors, migraine relieving devices, etc.

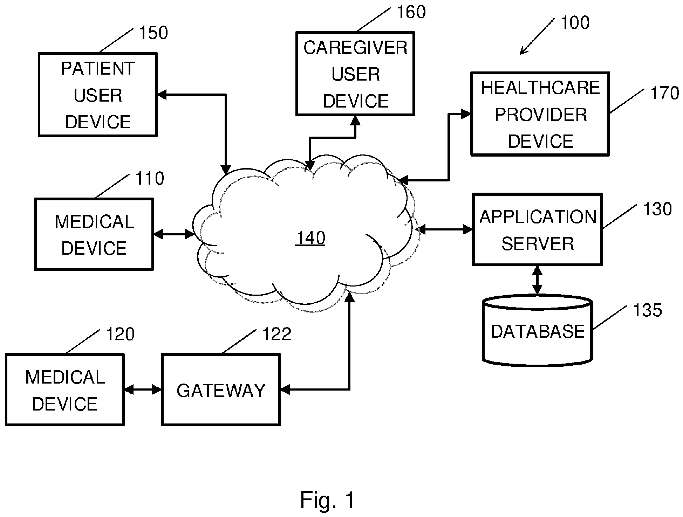

Reference is made to Fig. 1, which schematically illustrates a system 100, according to embodiments of the invention. System 100 may include one or more of medical devices 110 connectable to a network 140, e.g., the internet, and medical devices 120, each connectable to network 140 through a gateway 122. Each of medical devices 110 and 120 may be associated with a patient and with a usage schedule. Medical device 110 and medical device 120 together with gateway 122, may collect usage data and device status data and send the collected data over network 140 to application server 130. Medical device 110 may include a communication module that may enable direct connectivity to network 140. For example, medical device 110 may include a Wi-Fi or cellular module that may enable direct Internet connectivity. Medical device 120 may not include a communication module that enables direct connectivity to network 140, e.g., due to cost, weight, energy or other reasons. Instead, medical device 120 may connect to network 140 through gateway 122. For example, medical device 120 may include a communication module that enables short-range connectivity, e.g., Bluetooth or BLE, to a gateway 122. Gateway 122 may also include short-range connectivity module and a direct connectivity module that enables direct connectivity to network 140. Thus, gateway 122 may obtain communications from medical device 120 and may forward the information obtained from medical device 120 to any required module connected to network 140, or may receive information from any module connected to network 140 and forward the obtained information to medical device 120. Gateway 122 may be for example a mobile phone, or a dedicated of hardware module (for example, a Raspberry Pi device, etc.).

Application server 130 may also obtain data from a computer device 170 associated with the healthcare provider (e.g., physician, nurse, physiotherapist, rehabilitation professional). For example, healthcare provider device 170 may send patient profile, including medical information related to the patient, information regarding treatments to other diseases of the patient, and prescriptions or usage schedule for medical devices 110 and 120, to application server 130. The obtained data as well as other data such as device parameters, model parameters etc., may be stored in database 135.

Application server 130 may calculate a predicted adherence score based on for example the usage schedule and the device status data, device data, patient data and other information related to the medical treatment, and may perform a root cause analysis for detecting the reason for non-adherence by correlating the actual adherence score and the device data, patient data and other information related to the medical treatment, as disclosed herein. Application server 130 may provide alerts and adherence reports. For example, application server 130 may send non-adherence alerts and reports to medical devices 110 and 120, to patient user device 150, to a caregiver user device 160, healthcare provider device 170, etc.

Other system architectures may be used, for example according to some embodiments, medical devices 110 and 120, or patient user device 150 may calculate a predicted adherence score based on the usage schedule and the device status data and may perform a root cause analysis for detecting the reason for non-adherence by correlating the actual adherence score and the status data, as disclosed herein. Application server 130 may provide alerts and adherence reports. Additionally, various data items may be provided to system 100 by various components, depending on the system design. For example, some of the patient profile information may be provided by the healthcare provider through healthcare provider device 170, by the patient himself through patient user device 150, and/or by a caregiver through caregiver user device 160.

Networks 140 may include any type of network or combination of networks available for supporting communication between medical devices 110 and 120 (through gateway 122), application server 130, patient user device 150, caregiver user device 160, healthcare provider device 170, and databases 135. Networks 140 may include for example, wired and wireless telephone networks, the Internet and intranet networks, etc.

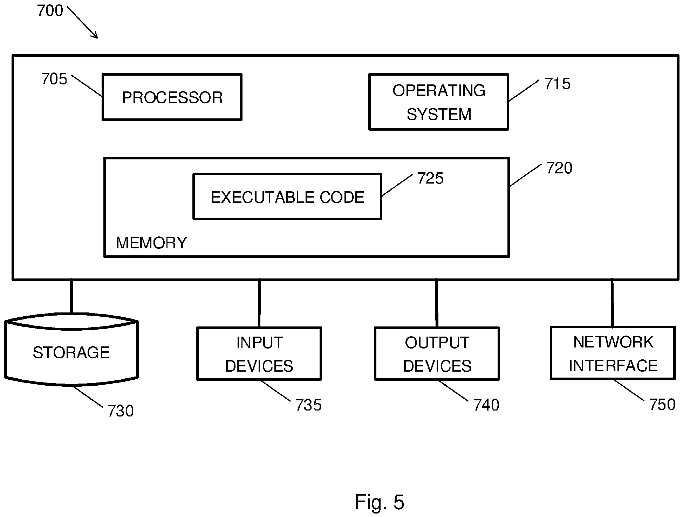

Each of medical devices 110 and 120, gateway 122, application server 130, patient user device 150, caregiver user device 160, and healthcare provider device 170 may be or may include a computing device such as computing device 700 depicted in Fig. 5. One or more databases 135 may be or may include a storage device such as storage device 730.

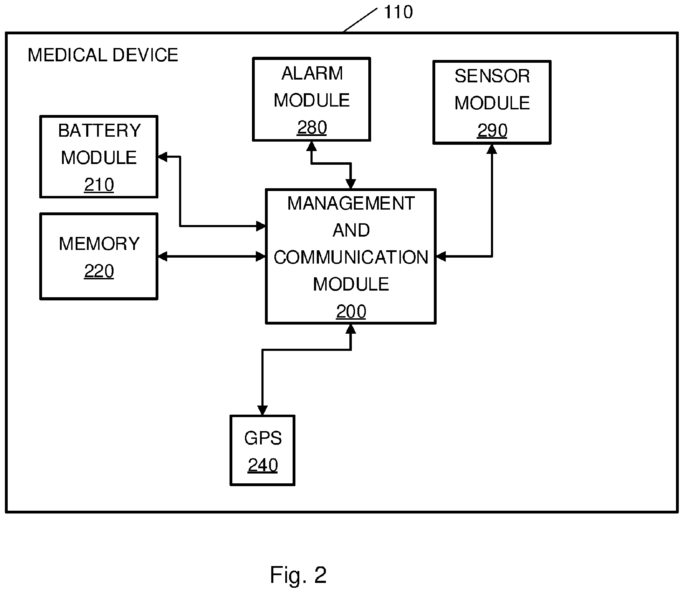

Reference is made to Fig. 2, which schematically illustrates medical device 110, according to embodiments of the invention. Medical device 110 may be or may include any type of medical device that is connectable to network 140. Medical device 110 may include some or all of the components disclosed herein. While Fig. 2 relates to a single medical device, in some application some of the functionalities described herein with relation to medical device 110 may be performed by medical device 120 and some by gateway 122.

Medical device 110 may include a management and communication module 200, which may gather device data, e.g., device general status data, and device medical data that may be relevant to adherence, from components of medical device 110 and send the adherence data to application server 130. Management and communication module 200 may also obtain data relevant to adherence from for example application server 130, healthcare provider device 170, patient user device 150 and caregiver user device 160. For example, management and communication module 200 may obtain:
- battery status from battery module 210,
- usage data, log files, firmware version and device configuration data, usage schedule and patient profile, from memory 220, and
- location data from global positioning system (GPS) 240.
Other parameters may be used.

For example, when medical device 110 or 120 is used, medical device 110 or 120 may send data to the cloud, e.g., to application server 130. For example, a remote blood pressure device may send the blood pressure result to application server 130 and thus application server 130 may have a record of the usage of the blood pressure device. Log files may include error logs as well as other information. For example, log files may include a fine-grained description of the way medical device 110 or 120 was used, including a record of every action the user has performed, action start time and end time, record of retries etc.

Medical device 110 or 120 may include a sensor module 290, that may serve the function of medical device 110 or 120 and at the same time may be used to sense usage of medical device 110 or 120. In some embodiments, a sensor module 290 may include sensors dedicated to sense usage of medical device. For example, sensor module 290 may include ultrasound sensors (e.g., in a pregnancy ultrasound device), radar or laser (e.g., for sensing vital signs remotely), light sensors (e.g., in wearables wristband), accelerometer and scales (e.g., assembled on orthopedic shoes, heat sensors, force sensors, and any other applicable sensors that may sense when the device is being used.

Medical device 110 or 120 may include an alarm module 280 for providing alarms, for example, when non-adherence is expected. Alarm module 280 may include one or more of a display, a light-emitting diode (LED), a loudspeaker or buzzer and a vibration motor for providing alarms in the form of blinking light, sound and/or vibration.

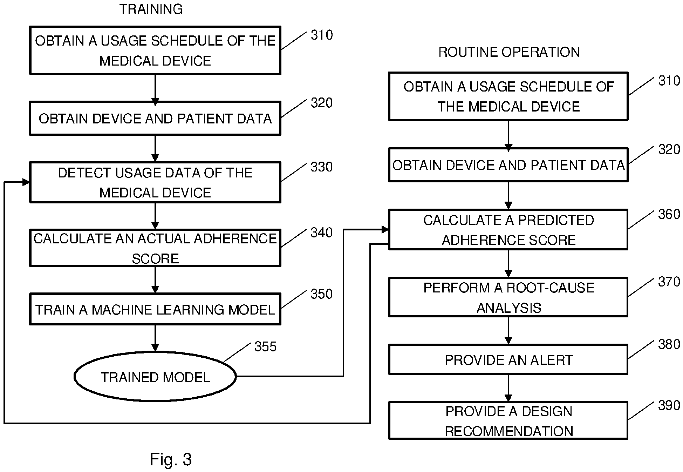

Reference is made to Fig. 3, which is a flowchart of a method for calculating predicted adherence, according to embodiments of the invention. An embodiment of a method for calculating predicted adherence may be performed, for example, by the systems shown in Figs. 1, 2 and 5, but other hardware may be used. Embodiments of the method for calculating predicted adherence may be divided into a training phase and a routine operation phase. In the training phase, a machine learning model may be trained to predict adherence to a medical device, and in the routine operation phase, the trained model may be used to predict adherence as disclosed herein.

The training phase may include generating training data set (operations 310-340) and training a model (operation 350). In operation 310, a usage schedule of the medical device may be obtained. The usage schedule may be determined for example, by a healthcare provider, e.g., a physician, a physiotherapist etc. The usage schedule may be provided for example, from healthcare provider device 170 or from medical devices 110 and 120 (through gateway 122). The usage schedule may include a prescription for using the medical device. The usage schedule may include, for example, actions that are needed to be performed by the device and timing of these actions. A usage schedule may also include configuration of the device, a required intensity, cycles of a repetitive action, duration of the activity of the device, etc. The usage schedule may include margins or deviations from the schedule that are still acceptable, or a range of required usage. For example, usage schedule of orthopedic or phsyiotheraputics shoes may include the time of day and duration of use (or a range of acceptable duration of usage) of the orthopedic or phsyiotheraputics shoes as determined by healthcare provider, e.g., a physiotherapist. For example, a usage schedule for orthopedic or phsyiotheraputics shoes may specify that the patient should wear the shoes for an hour every day, with margins of plus or minus 10 minutes every day.

In operation 320, device status data of the medical device, as well as patient data and other information related to the medical treatment may be obtained. The obtained data may include some or all of the following example data, or other data:
- Events log including a record of measurements performed by the medical device, treatments provided by the medical device and device status data. The data may include start time and end time of the treatments and measurement and success or failure of treatment and measurement (e.g., a record of a treatment or measurement that has started but was not completed).
- Patient medical background profile, including for example blood pressure, cardiovascular measurements, prescribed medications, age, gender, etc.
- Patient follow-up input. The patient follow-up input may be entered by the patient using a device-specific application (for example using patient user device 150 or medical devices 110 and 120) or by caregivers with for example an electronic health record system (for example using caregiver user device 160 or medical devices 110 and 120). The patient follow-up input may include for example a post-treatment patient questionnaire with a ranking of satisfaction from the device usability, current pain level, stress level, sensitivity to light, stress level, etc.
- Medical device status data, including for example, vendor specific configuration, firmware version, geographic location, battery charge status or level, device alarms etc. For example, the medical device status data may be detected by the medical device, e.g., medical devices 110 and 120 (with gateway 122) and sent over the internet to application server 130.
- Device log files, including operational and error log files. For example, device log files may be recorded and sent by medical devices 110 and 120 (with gateway 122) to application server 130.
- Environmental information of the medical device including for example, localization, temperature, humidity, moisture, gyroscope, climate, etc. For example, medical devices 110 and 120 may include a GPS (e.g., GPS 240) that may provide a location of the medical device. Other methods for obtaining the location of the medical device may be used. Medical devices

110 and 120 may include sensors and a sensor module 290 for measuring environmental conditions such as temperature, humidity, moisture, gyroscope, etc. Additionally or alternatively, environmental information may be obtained from patient user device 150, e.g., from a smartphone of the patient. For example, many smartphones include a GPS, temperature sensors, etc., which may provide location, temperature as well as other measurements of environmental conditions. In some embodiments some environmental conditions may be obtained from other sources over network 140, e.g., general climate data in the area of the medical device may be obtained over the Internet from weather websites.
- Patient and caregiver questionnaires, filled in for example in an application on patient user device 150, care giver user device 160 or healthcare provider device 170. The questionnaires may include data related to level of satisfaction of the patient and caregiver from the medical device, the treatment plan, level of involvement of the caregiver, level of caregiver satisfaction with the patient, etc.
- Time of usage and/or prescribed usage - for example the time in the day may influence adherence due to possible problem to adhere at work or at home. The day in a week may influence adherence due to for example problem to adhere during weekends. The date in the year may influence adherence due to for example problem to adhere during summer vacation, on school time, or on holidays.
- Time of appointments with the healthcare provider - a patient may adhere more to the prescribed medical device shortly before and shortly after medical appointments.
- Total duration of usage - the duration of usage may influence adherence in both directions. For example, if a user uses a device for a long time e.g., a year, and does not see any improvement, the user may adhere less to the device. However, if the patient or the caregiver notice a clear improvement, they may adhere more to preserve the good results, or may adhere less believing that they don't need the device anymore.

The obtained data may be unified and normalized, e.g., to a required scale. For example, different types of medical devices may use different formats for reporting the same type of data. Thus, the obtained data may be unified to a single standard. For example, some devices may report the number of failed attempts, while other will report a list of attempts to use the device in a log file, and the number of failed attempts may be deduced from the log files. The obtained data may be enriched, for example by deriving more useful and standard information out of the obtained data. For example, the treatment length may be calculated based on the raw data, for example by subtracting treatment start time from treatment end time, a number of failed attempts may be counted, time to next schedule (e.g., one hour left before the next needed usage), etc.

For example, the following example data items (also referred to as features) or other data may be obtained or calculated (e.g., some features may be calculated and added by aggregation or application of a mathematical function over combinations of measured or reported features):
- Device specification
   ∘ Weight.
   ∘ Dimensions.
   ∘ Hardware modules (BLE, battery, Wi-Fi modules, etc.), their manufacturers and technical specifications.
- Device usage scenario:
   ∘ Wearable, implantable, stationary, etc., each usage scenario may be coded, e.g., each device usage scenario may be associated with a value.
- Device configuration:
   ∘ Firmware version and other generic device parameters such as device name, time-zone settings, network timeout, number of retries before deciding the network is down, network settings, internet protocol (IP) address etc.
   ∘ Vendor-specific configuration parameters, including, for example calibration parameters, preferences settings etc.
- Device status:
   ∘ Location (including absolute location as well as district and country of usage).
   ∘ Battery charge level.
   ∘ Failed attempts to use.
   ∘ Length of usage.
   ∘ Activity logs.
   ∘ Error logs.
- Time of usage and/or prescribed usage:
   ∘ Time of day
   ∘ Day in a week
   ∘ Date in a year
- Trend in time:
   ∘ Time of appointments with the healthcare provider.
   ∘ Total duration of usage.
- Severeness of disease (e.g., coded relative to a scale).
- Other patient attributes and conditions that may affect adherence (e.g., attention deficit hyperactivity disorder ADHD, pregnancy).
- Demographic parameters that may affect adherence (e.g., age, gender etc.).
- Patient location, e.g., as determined according to the location of patient user device 150, e.g., by an application executed by a mobile device of the user.:
   ∘ Local.
   ∘ District.
   ∘ Country.
- Caregiver
   ∘ Level of involvement.
   ∘ Level of caregiver satisfaction with the patient, including whether the caregiver conveys this satisfaction to the patient.

In some embodiments, dimension reduction may be performed, e.g., using autoencoders, to transform the feature space of hundreds of features to two-dimensional (2D) or three-dimensional (3D) feature representation for customer visualization and for clustering. Feature engineering may be used to transfer the time series data of usage events to process related events as compliance may be related to treatment stage or condition severity.

In operation 330, actual usage data of the device may be detected. For example, the actual usage data may be detected by medical devices 110 and 120, and sent over network 140 to application server 130. Usage data may be detected by the software operating on, controlling or executed by medical devices 110 and 120, with the aid of sensors and/or may be deduced from the obtained log files.

In operation 340, an actual adherence score may be calculated for example by matching or comparing the usage schedule and the usage data. Thus, labeled data may be generated, including input data (as provided in operations 310 and 320), and corresponding output data calculated in operation 340. One or more adherence scores may be calculated on a daily, weekly, monthly basis, etc. For example, if a user has been prescribed to wear orthopedic or phsyiotheraputics shoes for an hour every day, and the user wears the orthopedic or phsyiotheraputics shoes for 50 minutes on Monday, then the daily adherence score may equal 50/60=0.83, or if the user wears the orthopedic or phsyiotheraputics shoes for one hour only on Monday to Friday, by comparing the usage schedule to the actual usage, the weekly adherence score may equal 5/7=0.71. The adherence score may take into account other parameters if applicable, such as adherence to a time of the day of use, allowed ranges of duration of use, required intensity versus used intensity, etc. A formula for calculating adherence score may be provided per device or device type, per prescription, per disease, etc.

In operation 350, one or more machine learning models 355 may be trained to predict adherence using the labeled data provided in operation 340. The model 355 may obtain as a set of input variables, predictors or independent variables the status data of the medical device, as well as patient data and other information related to the medical treatment, e.g., as provided in operation 320, and the usage schedule of the medical device obtained in operation 310. The target, outcome variable or dependent variable of model 355 may be the adherence score (e.g., the actual adherence score in the training phase). Model 355 may adjust weights and other model parameters based on the independent variables and the associated actual adherence score as calculated in operation 340.

The machine learning models 355 may include supervised learning models such as neural networks, regression models, decision trees, random forest, k-nearest neighbors (KNN), etc. For example, model 355 may be trained using data collected in operations 310 - 330 labeled in operation 340. The training data set may be collected from a plurality of medical devices and patients, as may be required by the application, e.g., operations 310-340 may be performed for a plurality of medical devices and patients. The training data set may include a list of instances, where each instance may include of the patient usage schedule, the device status, patient attributes, device and patient locations, device activity log, and error log, and other data items or features as disclosed herein. In addition, additional features may be calculated and added to the training dataset, e.g., by aggregation or application of a mathematical function over combinations of existing features. In some embodiments, a subject matter expert may label every instance in the training data set with an adherence score or provide a rule specifying how to label an instance with an adherence score (to be used in operation 340). The training process for predicting the adherence score may use the labeled data set to gradually refine a model for predicting the adherence score. Standard machine learning frameworks and algorithms may be used to build the model. The training process (e.g., for the supervised learning models) may continue until model 355 achieves a desired level of accuracy on the training data set.

According to some embodiments, operation 350 may be a part of a training phase or training session. According to some embodiments, operation 350 may be a one-time procedure initiated for each new patient, new device or new prescription, to adjust the machine learning model to predict the specific non-adherence. In some embodiments, operation 350 may be repeated periodically, e.g., once a day, once a week once a month, etc., to update the model according to changes in patient behavior that may occur over time. According to some embodiments, operation 350 may be initiated also when the accuracy of the model is not good, for example, if predicted adherence score previously calculated in operation 360 for a set of input data does not equal the actual adherence score for the same data, or if the precision is below a threshold.

In some embodiments, more than one model 355 (e.g., more than one type of machine learning mode) may be trained and evaluated, and the best performing model may be selected. Model evaluation may be done using any applicable method such as measuring precision and recall, using k-fold cross validation, etc. In some embodiments the machine learning model or models may be specific to a type of medical device, specific to a disease, specific to a combination of diseases, specific to patient demographics, specific to a particular patient history, or even patient specific.

According to some embodiments, if the system detects that the machine learning model (or that none of the machine learning models if more than one machine learning models is used) is unfit for the patient, e.g., if the predicted adherence score is not close to the actual adherence score, or if the levels of precision and recall are too low, the system may disable the adherence prediction and may provide a notification to the user, e.g., the patient, the caregiver and the manufacturer. During routine operation, usage schedule of the medical device may be obtained (operation 310) and device status data and/or patient status data, as well as other features may be obtained or calculated (operation 320). In operation 360, a predicted adherence score may be calculated using the model 355 generated in operation 350 and based on the usage schedule and the device status data, and optionally on other data items obtained in operation 320. In some embodiments, calculating the predicted adherence is performed using a machine learning model, e.g., a trained machine learning model 355 such as neural networks, regression model, decision tree, random forest, KNN, Markov decision process, etc.

In operation 370, root cause analysis may be performed in case of predicted non-adherence (e.g., predicted non-adherence below a threshold) or in case of actual non-adherence (actual adherence score below a threshold). For example, root cause analysis may be performed by correlating the adherence score with one or more of the input variables, predictors or independent variables. A root cause may relate to one or more features that may be related to or associated with an actual or predicted non-adherence. In some embodiments, any or all of the following measures may be used to suggest potential root causes: regression weights, correlation coefficients, predictive power score, local interpretable model-agnostic explanations (LIME) score, Shapley additive explanations (SHAP) score, or other feature explainers.

According to some embodiments, root cause analysis may be performed per patient, per disease, per device type, etc. Calculating adherence per device type may provide insights as to why patients adhere to certain devices more than to others, despite similarities.

According to some embodiments, after analyzing a big amount, e.g., hundreds of different medical device products, enough non-adherence root causes may be determined. The common root causes may be addressed in the design of new medical devices. Examples for such root causes may include for example:

Correlation of adherence scores to weight of the device - may provide a recommendation of maximum weight per type of device before adherence decreases.

Correlation of adherence scores to lifespan of a battery - may provide a recommendation of minimum lifespan of a battery per type of device (wearable, implantable).

Correlation of adherence scores to range of BLE (or any RF) module - may allow recommending the best BLE device manufacturer.

In operation 380, an alert may be provided in case that non-adherence is expected. For example, if the predicted adherence score calculated in operation 360 exceeds a threshold, e.g., is below a threshold or above a threshold depending on the application. Stakeholders, e.g., the patient, the caregiver, the healthcare provider or any other relevant person may be notified of the predicted non-adherence in any applicable manner, e.g., by sending a notification to medical device 110 or 120 (via gateway 122), to patient user device 150, to care giver user device 160 or to healthcare provider device 170, or any applicable device. The alert may include an audible alert, a visual alert, a message (e.g., an SMS or WhatsApp message), etc. The alert may include calling the patient and/or his caregiver.

According to some embodiment, the non-adherence prediction notification may include details about the root cause of non-adherence, e.g., the sequence of events leading to the prediction. A textual explanation may be generated based on the root cause so that the doctor and/or patient may be able to understand the reason and act upon it. For example, a textual explanation may include "We notify because you are out of home now, 30 minutes away from home, and treatment is due in one hour".

According to some embodiments, effectiveness of the alerts and notification may be analyzed. The lead time of the notification may be adjusted accordingly, using for example a reinforcement learning algorithm. According to some embodiments, alerts and notifications may be sent also after the prescribed time for using the device, in case it is detected that patients may be late at times but do adhere eventually. According to some embodiments, the real influencers on adherence or the best channels for sending alerts and notifications may be detected (e.g., using root cause analysis) to provide more effective notifications with less overhead.

According to embodiments of the invention, effectiveness of notifications may be analyzed and channels for providing notifications may be improved based on results of the analysis. For example, notifications may be provided to users in a variety of notification channels. Data related to user actions following the notifications may be collected and analyzed. For example, adherence data may be collected and analyzed following adherence notifications. The collected data may be used to optimize the delivery channel of the notification, for example, the optimized notification channel parameters may include who gets the notification, the timing of sending the notification, and the channel for providing the notification. Analyzing the effectiveness of notifications may include correlating parameters of the notification channels and the actions performed by the users to obtain effectiveness of each of the plurality of channels. Analyzing the effectiveness of notifications may be performed with relation of adherence, however, this is not limiting and effectiveness of other notifications that are related to measurable outcomes (e.g., measurable actions the user has to take following the notifications) may be analyzed. Following the analysis, recommendations as to which channel parameters provide the highest effectiveness may be provided.

In operation 390 a recommendation for a change in the design of the device may be derived based on the root cause of the non-adherence and provided to a user. For example, correlation between non-adherence and the weight of the device may create a recommendation of maximum weight per type of device before adherence drops, correlation between non-adherence and the lifespan of the battery of medical device 110 or 120 may create a recommendation of minimum battery lifespan per type of device (e.g., wearable, implantable, etc.), correlation between non-adherence and the range of BLE (or any RF) module may allow recommending the best BLE device manufacturer, etc.

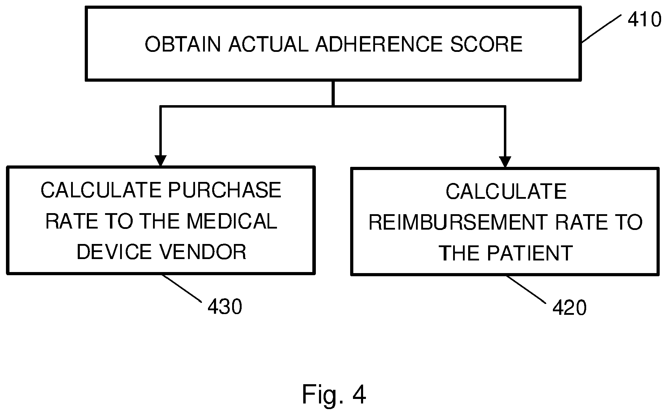

Reference is made to Fig. 4, which is a flowchart of a method for adjusting reimbursement rate and purchase rate of medical devices based on adherence level, according to embodiments of the invention. An embodiment of a method for adjusting reimbursement rate and purchase rate of medical devices based on adherence level may be performed, for example, by the systems shown in Figs. 1 and 5 but other hardware may be used. Adjusting reimbursement rate of medical devices based on adherence level may provide an incentive to the patient to use the medical device as prescribed. Adjusting purchase rate of medical devices based on adherence level may provide an incentive to the provider of the medical device to provide a medical device that is easy to use.

In operation 410 actual adherence scores may be obtained. For example, actual adherence scores over time of a specific patient to a specific device, e.g., as calculated in operation 340 may be obtained. For example, actual adherence scores may be obtained periodically (e.g., on a monthly basis, daily basis, etc.) or in real time upon an ad hoc decision that adherence score is low, e.g., on application server 130 or on a payer cloud. In some embodiments, root causes for non-adherence may be sent as well.

In operation 420, a reimbursement rate to the patient may be calculated or determined based on the adherence scores. For example, an average adherence score over a time period may be calculated. The reimbursement rate to the patient may be linear or proportional with the average adherence score or may be nearly linear using a step function. Other functions for calculating the reimbursement rate based on the adherence score may be used. According to some embodiments, the root cause for non-adherence may mitigate the effect of non-adherence on the reimbursement rate in case the root cause is device related.

In operation 430, a purchase rate or cost paid to the medical device vendor for the medical device may be calculated or determined based on the adherence scores. For example, an average adherence score over a time period may be calculated. The purchase rate or cost may be linear or proportional with the average adherence score or may be nearly linear using a step function. Other functions for calculating the purchase rate or cost based on the adherence score may be used. According to some embodiments, the root cause for non-adherence may mitigate the effect of non-adherence on the purchase rate or cost in case the root cause is patient related.

Embodiments of the invention may be utilized for other applications that require adherence, for example embodiments of the invention may be used for improving adherence to responding to questionnaires. Some medical procedures, and especially medical procedures that are under research, require gathering information from the patient and/or caregiver at predetermined time intervals relatively to performing a procedure. For example, a patient may be prescribed to use a medical device and may have to provide information e.g., by answering questionnaires while being treated, an hour after treatment, a day after treatment and one week after treatment. These questionnaires may allow companies to collect important patient data for medical research over big data to find correlations between cause and effect. In addition, new medical device regulation (MDR) may require manufacturers of medical devices to monitor the usage and efficacy of the device in the market. One popular method to perform this is by using patient questionnaires.

However, sending the routine questionnaire repeatedly may cause patients and caregivers to adhere less to responding to the questionnaires. The more frequent the questionnaires, the faster patients may stop responding to questionnaires. According to embodiments of the invention, adherence to questionnaires, e.g., the present or ratio of filled questionnaires relatively to the total number of questionnaires provided, may be analyzed and improved. For example, data regarding filling of questionnaires may be collected, for example from the device that is used to present the questionnaires to the user and for filing up the questionnaires, e.g., patient user device 150 and/or caregiver user device 160. The data may include adherence to the questionnaires, e.g., the ratio of filled questionnaires to the total number of questionnaires and/or adherence to a specific questionnaire suggesting whether the questionnaire was filled or not. The collected data may further include data regarding questionnaire types and questionnaire parameters, including for example, the relative time (e.g., time relatively to treatment) and absolute time (e.g., time in the day, day in the week, date, etc.) of when the patient or other user is requested to answer the questions, number of questions in the questionnaires, the time required for the user to fill the questions, in what question (# in questionnaire or question content) the patient abandoned the questionnaire, etc. In addition, different questionnaire programs may be monitored, e.g., alternating versus fixed questionnaires, different lengths of questionnaires, e.g., alternating length of questionnaire, various incentives (e.g., loyalty programs), etc. Adherence data for the questionnaires may be collected and analyzed with relation to the input parameters, to find correlations between any of the above listed parameters and adherence levels. Thus, root causes for adherence and for non-adherence to questionnaires may be detected. Based on the calculated correlations and the detected root causes, recommendations may be provided as to which questionnaire programs (e.g., questionnaire types or parameters) provide the highest adherence levels. In some embodiments, a questionnaire program may be altered based on the detected root causes in order to increase adherence.

Fig. 5 illustrates an example computing device according to an embodiment of the invention. Various components such as medical devices 110, medical devices 120, gateways 122, application server 130, healthcare provider device 170, patient user device 150, caregiver user device 160, and other modules, may be or include computing device 700, or may include components such as shown in Fig. 5. For example, a first computing device 700 with a first processor 705 may be used to calculate predicted adherence scores and root causes for non-adherence.

Computing device 700 may include a processor 705 that may be, for example, a central processing unit processor (CPU), a chip or any suitable computing or computational device, an operating system 715, a memory 720, a storage 730, input devices 735 and output devices 740. Processor 705 may be or include one or more processors, etc., co-located or distributed. Computing device 700 may be for example a workstation or personal computer, or may be at least partially implemented by one or more remote servers (e.g., in the "cloud").

Operating system 715 may be or may include any code segment designed and/or configured to perform tasks involving coordination, scheduling, arbitration, supervising, controlling or otherwise managing operation of computing device 700, for example. Operating system 715 may be a commercial operating system. Memory 720 may be or may include, for example, a Random Access Memory (RAM), a read only memory (ROM), a Dynamic RAM (DRAM), a Synchronous DRAM (SD-RAM), a double data rate (DDR) memory chip, a Flash memory, a volatile memory, a non-volatile memory, a cache memory, a buffer, a short term memory unit, a long term memory unit, or other suitable memory units or storage units. Memory 720 may be or may include a plurality of, possibly different memory units.

Executable code 725 may be any executable code, e.g., an application, a program, a process, task or script. Executable code 725 may be executed by processor 705 possibly under control of operating system 715. For example, executable code 725 may be or include an application calculate predicted adherence scores and root causes for non-adherence. In some embodiments, more than one computing device 700 may be used. For example, a plurality of computing devices that include components similar to those included in computing device 700 may be connected to a network and used as a system.

Storage 730 may be or may include, for example, a hard disk drive, a floppy disk drive, a Compact Disk (CD) drive, a CD-Recordable (CD-R) drive, a universal serial bus (USB) device or other suitable removable and/or fixed storage unit. In some embodiments, some of the components shown in Fig. 5 may be omitted. For example, memory 720 may be a non-volatile memory having the storage capacity of storage 730. Accordingly, although shown as a separate component, storage 730 may be embedded or included in memory 720.

Input devices 735 may be or may include a mouse, a keyboard, a touch screen or pad or any suitable input device. It will be recognized that any suitable number of input devices may be operatively connected to computing device 700 as shown by block 735. Output devices 740 may include one or more displays, speakers and/or any other suitable output devices. It will be recognized that any suitable number of output devices may be operatively connected to computing device 700 as shown by block 740. Any applicable input/output (I/O) devices may be connected to computing device 700 as shown by blocks 735 and 740. For example, a wired or wireless network interface card (NIC), a modem, printer or facsimile machine, a universal serial bus (USB) device or external hard drive may be included in input devices 735 and/or output devices 740. Network interface 750 may enable device 700 to communicate with one or more other computers or networks. For example, network interface 750 may include a Wi-Fi or Bluetooth device or connection, a connection to an intranet or the internet, an antenna etc.

Embodiments described in this disclosure may include the use of a special purpose or general-purpose computer including various computer hardware or software modules, as discussed in greater detail below.

Embodiments within the scope of this disclosure also include computer-readable media, or non-transitory computer storage medium, for carrying or having computer-executable instructions or data structures stored thereon. The instructions when executed may cause the processor to carry out embodiments of the invention. Such computer-readable media, or computer storage medium, can be any available media that can be accessed by a general purpose or special purpose computer. By way of example, and not limitation, such computer-readable media can comprise RAM, ROM, EEPROM, CD-ROM or other optical disk storage, magnetic disk storage or other magnetic storage devices, or any other medium which can be used to carry or store desired program code means in the form of computer-executable instructions or data structures and which can be accessed by a general purpose or special purpose computer. When information is transferred or provided over a network or another communications connection (either hardwired, wireless, or a combination of hardwired or wireless) to a computer, the computer properly views the connection as a computer-readable medium. Thus, any such connection is properly termed a computer-readable medium. Combinations of the above should also be included within the scope of computer-readable media.

Computer-executable instructions comprise, for example, instructions and data which cause a general purpose computer, special purpose computer, or special purpose processing device to perform a certain function or group of functions. Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

As used herein, the term "module" or "component" can refer to software objects or routines that execute on the computing system. The different components, modules, engines, and services described herein may be implemented as objects or processes that execute on the computing system (e.g., as separate threads). While the system and methods described herein are preferably implemented in software, implementations in hardware or a combination of software and hardware are also possible and contemplated. In this description, a "computer" may be any computing system as previously defined herein, or any module or combination of modulates running on a computing system.

For the processes and/or methods disclosed, the functions performed in the processes and methods may be implemented in differing order as may be indicated by context. Furthermore, the outlined steps and operations are only provided as examples, and some of the steps and operations may be optional, combined into fewer steps and operations, or expanded into additional steps and operations.

The present disclosure is not to be limited in terms of the particular embodiments described in this application, which are intended as illustrations of various aspects. Many modifications and variations can be made without departing from its scope. Functionally equivalent methods and apparatuses within the scope of the disclosure, in addition to those enumerated, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is also to be understood that the terminology used in this disclosure is for the purpose of describing particular embodiments only, and is not intended to be limiting.

This disclosure may sometimes illustrate different components contained within, or connected with, different other components. Such depicted architectures are merely exemplary, and many other architectures can be implemented which achieve the same or similar functionality.

Aspects of the present disclosure may be embodied in other forms without departing from its spirit or essential characteristics. The described aspects are to be considered in all respects illustrative and not restrictive. The claimed subject matter is indicated by the appended claims rather than by the foregoing description. All changes which come within the meaning and range of equivalency of the claims are to be embraced within their scope.

The description also includes the subject matter of the following clauses:

### CLAUSES

1. A system for detecting adherence to a medical device, the system comprising:
   a memory;
   a processor configured to:
      obtain a usage schedule of the medical device;
      obtain device status data of the medical device; and
      calculate a predicted adherence score based on the usage schedule and the device status data.
2. The system of clause 2, wherein the status data includes at least one of: battery status, error logs and location of the medical device.
3. The system of any of clauses 1 and 2, wherein the status data includes battery charge status, wherein the processor is configured to calculate the predicted adherence score based on the battery charge status.
4. The system of any of clauses 1 to 3, wherein the status data includes location of the medical device, wherein the processor is configured to calculate the predicted adherence score based on the geographic location of the medical device.
5. The system of any of clauses 1 to 4, wherein the processor is configured to provide an alert in case the predicted adherence score exceeds a threshold.
6. The system of any of clauses 1 to 5, wherein the processor is configured to calculate the predicted adherence using a machine learning algorithm.
7. The system of clause 6, wherein the machine learning algorithm is one of patient specific, device type specific, or disease specific.
8. The system of any of clauses 1 to 7, wherein the processor is configured to:
   detect usage data of the medical device;
   calculate an actual adherence score by matching the usage schedule and the usage data; and
   perform a root cause analysis for detecting the reason for non-adherence by correlating the actual adherence score and the device status data.
9. The system of clause 8, wherein the processor is configured to calculate the actual adherence score based on allowed deviations from the usage schedule.

## Claims

1. A method for detecting adherence to a medical device, the method comprising:
using a processor:
obtaining a usage schedule of the medical device;
obtaining device status data of the medical device; and
calculating a predicted adherence score based on the usage schedule and the device status data.

2. The method of claim 1, wherein the status data includes at least one of: battery charge status, error logs and location of the medical device.

3. The method of any of the preceding claims, wherein the status data includes battery charge status, the method further comprising calculating the predicted adherence score based on the battery charge status.

4. The method of any of the preceding claims, wherein the status data includes location of the medical device, the method further comprising calculating the predicted adherence score based on the geographic location of the medical device.

5. The method of any of the preceding claims, comprising providing an alert in case the predicted adherence score exceeds a threshold.

6. The method of any of the preceding claims, wherein calculating the predicted adherence score is performed using a machine learning algorithm.

7. The method of claim 6, wherein the machine learning algorithm is one of patient specific, device type specific, or disease specific.

8. The method of any of the preceding claims, comprising:
detecting usage data of the medical device;
calculating an actual adherence score by matching the usage schedule to the usage data; and
performing a root cause analysis for detecting the reason for non-adherence by correlating the actual adherence score and the device status data.

9. The method of claim 8, wherein calculating the actual adherence score is based on allowed deviations from the usage schedule.

10. The method of claim 8, comprising:
providing a notification about non-adherence including the root cause of the non-adherence.

11. The method of claim 8, comprising:
providing a recommendation for a change in the design of the device based on the root cause of the non-adherence.

12. A system for detecting adherence to a medical device, the system comprising:
a memory;
a processor configured to perform the method of any of the preceding claims.

13. A computer readable medium comprising instructions which when executed by a processor in a computing system cause the processor to perform the steps of any of claims 1 to 11.
